# EUROPEAN PATENT SPECIFICATION

(11) **EP 3 442 367 B1**
(45) Date of publication and mention of the grant of the patent: **21.02.2024**
(21) Application number: 17781652.7
(22) Date of filing: 18.04.2017
(51) Int. Cl.: A41D 1/08, A41D 13/00, A63B 21/00, A41D 31/18

(54) **COMPRESSION GARMENT**
KOMPRESSIONSKLEIDUNG
VÊTEMENT DE COMPRESSION

(30) Priority: 14.04.2016 AU 2016901400
(43) Date of publication of application: 20.02.2019
(73) Proprietor: Symphony Holdings Limited, King's Road, North Point (HK)
(72) Inventor: NOONAN, Toni Elizabeth, Riverwood New South Wales 2210 (AU)
(74) Representative: Lawrence, Richard Anthony
(86) International application number: PCT/AU2017/050348
(87) International publication number: WO 2017/177285

(56) References cited:
- US-A1- 2008 083 055
- US-A1- 2013 326 785
- US-A1- 2016 015 545

## Description

### Field of the Invention

The present invention relates to compression garments and, in particular, to compression pants or tights and methods of providing same.

The invention has been developed primarily in respect of compression garments in the form of long pants or trousers extending from the waist of a user to past their knees and will be described hereinafter with reference to this application. However, it will be appreciated that the invention is not limited to this particular specific length, but lies within the length disclosed in the appended claims.

### Background of the Invention

Compression garments in the form of pants or tights have been long known. For example, US Patent No. 6,186,970 and 5,263,923 disclose the use of a strip of differential compression fabric or fabrics having a different stretchability to a base fabric. The strip extends from the waist of a user to the inside of the hamstrings and extending downwardly. For example, in the '970 patent, a pair of panels having different elasticity or stretchability in a longitudinal direction are aligned and where an intermediate panel is disposed having its warp or longitudinal axis disposed perpendicular or across the first two panels. This is disclosed to provide support for the garment wearer in use according to the `970 patent.

In US Patent Publication No. 2008/0120757, compression garments in the form of tights are provided. These use strips running from the waist down each leg from the hip and waist region to the inside of the thighs where they then extend substantially down the leg. This garment is said to assist in providing action which is supposed to draw the knee upward toward the waist to promote forward movement of the thighs. US Patent No. 8,296,864 provides a compression garment having strips running down each leg where the strips are adapted to have less longitudinal stretchability than a base material. These strips include apertures in various locations and extend from the waist to the ankles with the stated aim of stabilising the knee of a wearer in use.

US Patent No. 8,356,363 attempts to provide stability to a wearer of a compression garment in the form of short pants or bicycle shorts. Similarly to the above US patent the '363 patent discloses the use of a compression garment having elastic elements attached to the compression garment base to provide support. US Patent No. 8,533,864 discloses a compression garment having a fabric of less elasticity than the base material that is disposed on the inside. The base material is connected together or attached with the elastic fabric disposed intermediate forming a joint seam.

The document US2008/083055A1 discloses a compression garment.

Of all the aforementioned prior art, it is believed that improved stability can be achieved by configuring compression pants providing a tension against the twisting of the leg or knee or the lateral displacement or longitudinal stretching of the leg of a wearer.

### Genesis of the Invention

The genesis of the present invention is a desire to provide compression pants or tights having a predetermined tension against twisting of the leg or knee of a wearer or the lateral displacement or longitudinal stretching of the leg of a wearer, or to provide a useful alternative.

### Summary of the Invention

In accordance with an aspect of the present invention there is disclosed a compression garment extending longitudinally from a waist to at least a distal or lower end of the gastrocnemius or the soleus of each leg of a wearer, the garment comprising:
at least one panel of stretchable material stitched together by a plurality of seams to form a compression garment outer having a waist portion and a pair of leg portions wherein the compression garment outer includes an inside surface facing the user and an external surface , the panel having a warp or longitudinal stretch of between 150% to 225%;
at least one frictive element attached to the inside of the compression garment outer and extending from the waist to the distal or lower end of the gastrocnemius or to the soleus wherein the frictive element has a warp or longitudinal stretch of between 101% to 125%, the frictive element having a plurality of strips of predetermined constant or non-constant width;
wherein a first frictive strip extends from the waist about the tensor fasciae latae or sartorius muscle head down to the Sartorius muscle near a top of the vastus medialis, a second frictive strip extending from the waist about the gluteus medius around the vastus intermedius and converging with the first strip over the Sartorius muscle, and a third strip extending from the gluteus maximus at the waist adjacent the spine around the vastus lateralis to converge with the second frictive strip over the iliotibial tract whereby the converged second and third strips extend along the vastus intermedus and converge with the first strip at the sartorius muscle and extending along the rectus femoris a predetermined distance above the patella bifurcating along each side thereof, the frictive element extending a predetermined distance below the patella and around the tibia about the iliotibial tract joining at the distal or lower end of the gastrocnemius muscles or the soleus muscle.

It can therefore be seen that there is advantageously provided a compression garment having a frictive element advantageously disposed intermediate a compression garment base and the skin of a wearer so as to resist twisting of the knee or leg as well as longitudinal or lateral stretching or lateral displacement.

### Description of the Drawings

A preferred embodiment of the invention will now be described, by way of example only, with reference to the accompanying drawings in which:
Fig. 1 is a front view of a compression garment according to a first preferred embodiment;
Fig. 2 is a rear view of the garment of Fig. 1;
Fig. 3 is a right hand side view of the garment of Fig. 1 (left hand side equals mirror);
Fig. 4 is a front view of a compression garment according to a second preferred embodiment;
Fig. 5 is a rear view of the compression garment of Fig. 4;
Fig. 6 is a right hand side view of the compression garment of Fig 4 (left hand side equals mirror);
Fig. 7 is a front view of the compression garment according to another preferred embodiment;
Fig. 8 is a rear view of the garment of Fig. 7;
Fig. 9 is a right hand side view of the garment of Fig. 7 (left hand side equals mirror);
Fig. 10 is are various photographic views of the garment of Fig. 1 worn by a user;
Fig, 11 is a schematic diagram of a compression garment according to Fig. 1 without panels shown over the muscles of a wearer; and
Fig. 12 is a schematic diagram of the garment of Fig. 11 showing garment panels.

### Detailed Description

It will be appreciated that throughout the description of the preferred embodiments that like reference numerals have been used to denote like components unless expressly noted otherwise.

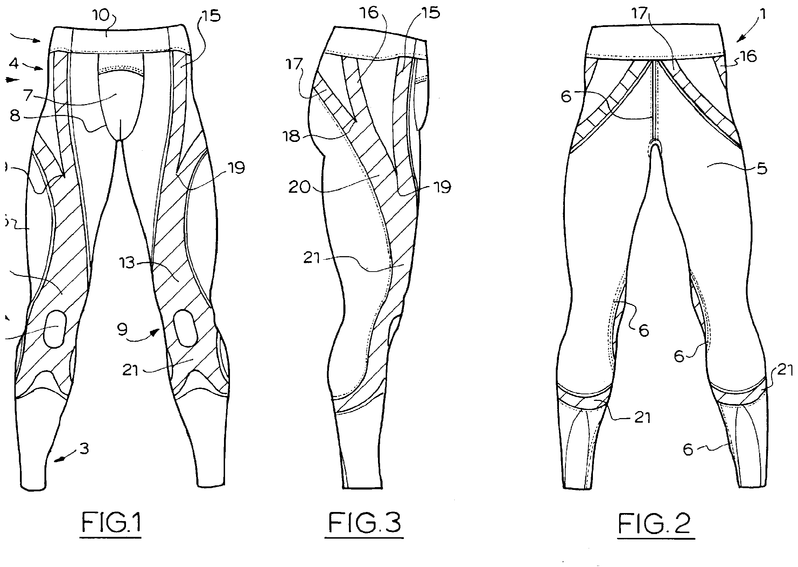

In Figs. 1-3, there is shown views of a compression garment 1 in the form of long pants or trousers. The compression garment 1 extends longitudinally from a waist 2 to a distal end 3 adapted to be disposed at least at the distal or lower end of the gastrocnemius (calf) muscle or soleus on each leg of a wearer. The compression garment 1 is formed from at least one panel (e.g. 5) of stretchable material. The panel(s) 5 or cut out(s) is joined or stitched together by a plurality of seams 6 to form the trouser compression garment 1.

In the embodiment of Fig. 1, a second panel of stretchable material is stitched into the panel 5 forming a crotch portion 7 from that second panel. The second panel is provided to account for the contour of the crotch region and is stitched by a seam 8 to the panel 5. However, it will be appreciated that the stitched seams are not limited to simple thread seams but can be glued, melted or welded amongst any preferred method of joining/attachment.

The compression garment 1 formed from the panels (e.g. 5 and 7) includes a waist portion 4 and a pair of leg portions 9. The panels of stretchable material 5 and 7 when stitched together with seams (e.g. 6 and 8) have the compression garment 1 with an inside surface intended to be contiguous with the skin of a user when worn thereby and an outer surface or external facing surface. In the preferred embodiment of Fig. 1, the fabric of the panels (e.g. 5 and 7) have a warp or longitudinal stretch value being an average of about 175%. It will be appreciated that the stretchability of the material forming the panels (e.g. 5 and 7) can be different to each other and is preferably in the range of 150% to 225% for longitudinal or warp stretch. A waist band 10 is provided about the opening of the waist portion 4 to elasticise the waist of the garment to maintain a fit on the user. The panels (e.g. 5 and 7) being formed from stretchable material naturally conform to the shape of a wearer.

A pair of frictive elements 12 and 13 are attached to the inside facing surface of the leg portions 9 forming the compression garment 1. It will be appreciated that the panels (e.g. 5 and 7) forming the compression garment 1 are termed "compression garment outer". The frictive elements 12 and 13 extend along each leg of the compression garment 1 from the waist to a portion of the leg near the distal or lower end of the gastrocnemius or soleus. Fig. 3 is a right side view of the compression garment of Fig. 1 showing frictive element 12 and it will be appreciated that the left side view is a mirror of the right side view. As such, only one side of the frictive element is described and the other side has a corresponding like frictive element 13 not shown in Fig. 3.

The frictive elements 12 and 13 are formed from a material having a warp or longitudinal stretch of between 110% and 125%. The frictive elements 12 and 13 are preferably formed from a non-woven material such as a natural or synthetic rubber or polymeric material. However, it will be appreciated that the frictive elements 12 and 13 can also be formed from impregnated fabrics or otherwise be provided with a rough surface to increase the friction between the frictive elements 12 and 13 and the skin of a user. In this regard, the co-efficient of static friction of the frictive elements on the skin of a user is preferably between 0.5 and 1.25. The co-efficient of static friction of the frictive elements 12 and 13 is selected so that when in contact with a wearer a resistance to movement across the surface of the skin of the frictive elements 12 and 13 is provided. In the case of a natural or synthetic rubber forming the frictive elements 12 and 13, it will be appreciated that the stretchability will be substantially isotropic, or substantially identical along and across each element. This is because in the preferred embodiment, the frictive elements 12 and 13 are made from synthetic rubber or natural rubber and are not woven so have a stretch substantially independent of direction, such as a direction of warp and weft. As shown in Figs. 1-3 and described below, the frictive elements 12 and 13 are of a non-constant width and are attached to the inside facing surface of the compression garment outer formed from the panels (e.g. 5 and 7) stitched or otherwise attached together. The frictive elements 12 and 13 are stitched to the panel(s) (e.g. 5) in the preferred embodiment, however, it will be appreciated that other means of attaching the frictive elements 12 and 13 to the panel 5 can be used. For example, frictive elements 12 and 13 may be glued to the panel(s) or they may be welded or melted to the panel(s) by the application of heat. Similarly, means of joining panels other than stitching can be used, such as welding. It will be appreciated that any preferred means of attaching the frictive elements 12 and 13 to the panel 5 forming the compression garment outer can be used as desired provided the location of the frictive elements 12 and 13 relative to the panel 5 forming the compression garment outer remains fixed.

With respect to frictive element 12, and noting frictive element 13 on the left hand side of a garment 1 is symmetrically identical to the right hand side, only the frictive element 12 on the right hand side will be described. Muscle groups are referred to as a locational indication of the nearest skin surface only, and it will be appreciated that there may be muscles or tissues disposed between those muscles referred to for this purpose and the compression garment itself.

A first frictive strip 15 extends from the waist 2 of the garment 1 from about the tensor fasciae latae or sartorius muscle head. The first strip 15 extends downwardly approximately along the Sartorius muscle to near a distal end of of the vastus medialis, a second frictive strip 16 also extends from the waist 2 of the compression garment 1 from about the gluteus medius or a posterior portion of the gluteus medius, and across the iliotibial tract or tensor fasciae latae (or preferably across the vastus medius), to converge with the first frictive strip 15 approximately over a central portion of the Sartorius muscle or a central portion of the rectus femoris, or approximately above the distal end of the adductor longus or pectineus muscle (or preferably the vastus medius) on an anterior side of the leg.

A third frictive strip 17 extends approximately from an anterior region of the Gluteus maximus at the waist 2 of the compression garment 1 adjacent the spine. The third frictive strip 17 extends approximately parallel or subparallel to the gluteus maximus muscle fibres (or preferably the vastus lateralus fibres) before converging with the second frictive strip approximately over the iliotibial tract near its head with the tensor fasciae latae or gluteus maximus muscles or the tendons thereof. The converged second and third frictive strips 16 and 17 further extend approximately parallel or subparallel to the vastus lateralis muscle fibres (or in an alternate embodimentthe vastus intermedus fibres) and converge with the first strip near the satorius muscle as described previously. Preferably the first, second, and third frictive strips are approximately equal or at least reasonably comparable in width prior to their respective first points of convergence.

The converged frictive strips 15, 16, 17 extend downward as a single frictive strip 21 approximately over a central portion of the rectus femorus, then approximately over a distal portion of the vastus medialis, and down past the knee of a wearer (preferably extending along the rectus femorus). Preferably the combined frictive element 21 has an approximate width of at least two of the individual frictive strips at its proximal end, and preferably widens towards the patella 22. The combined frictive element 21 bifurcates above and runs around the patella 22, approximately along the iliotibial tract towards its attachment near gerdy's tubercle on the outside of the knee, approximately along and/or over the Sartorius muscle on the inside of the knee, and re-converging below the patella 22. A predetermined distance below the knee, which may substantially coincide with the aforesaid point of re-convergence of the combined frictive element 21 below the patella 22, the converged frictive material 21 bifurcates or wraps around the tibia, preferably approximately at the distal or lower end of the gastrocnemius muscleto the distal end of the soleus muscle. Preferably, the two frictive strips that bifurcate around the tibia re-converge on the posterior side of the leg.

In this way, the frictive elements 12 and 13 are discouraged from moving relative to adjacent skin. This has the advantageous effect of providing a tension against movement of the garment 1 over the skin. Frictive strips 15, 16 and 17 act singularly and when converged to anchor the frictive strip to the skin of the wearer underneath the panel(s) (e.g. 5) forming the compression garment outer. It is believed by the inventors that disposing the frictive elements 12 and 13 substantially about the muscle groups as shown, that a tension is provided against twisting or rotation of the knee and also against longitudinal separation or lateral movement of the leg or knee joint. Further, it is also believed that the proprioceptors of the adductor, quadriceps, gluteal and iliotibial musculoskeletal groups, and golgi tendon organs and associated joint receptors thereof, are stimulated by the frictive elements 12 and 13, as well as the gastrocnemius muscle proprioreceptors ..

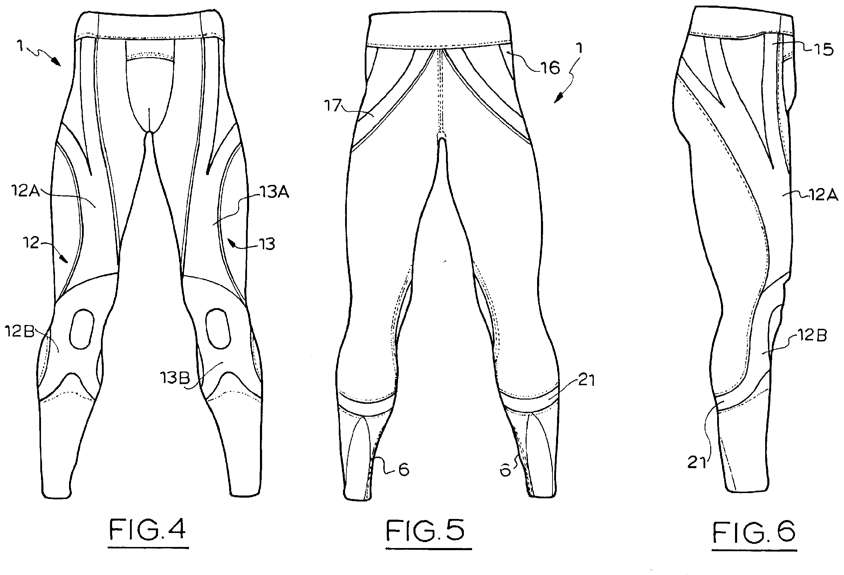

Referring to Figs. 4-6, there is shown a second preferred embodiment of the compression garment 1. In the second preferred embodiment, the frictive elements 12 and 13 are each formed from two parts. Particularly, and noting that the left hand frictive element 13 is a mirror image of the right hand frictive element 12, the frictive elements are formed from two separate components that are disposed contiguously or closely adjacent each other. In this way, frictive strips 12 and 13 are formed by upper frictive strips 12A and 13A respectively, and lower frictive strips 12B and 13B respectively. Frictive strips 12A and 13A include their respective first, second and third frictive strips 15-17, and the lower portion of the frictive strips 12B and 13B extend from above the knee downwardly. It will be appreciated that any preferred number of elements can be used to form frictive elements 12 or 13 and that each of the elements forming frictive elements 12 or 13 need only be disposed contiguous with or closely adjacent each other so as to provide a substantially continuous frictive element extending from the waist to below the knees.

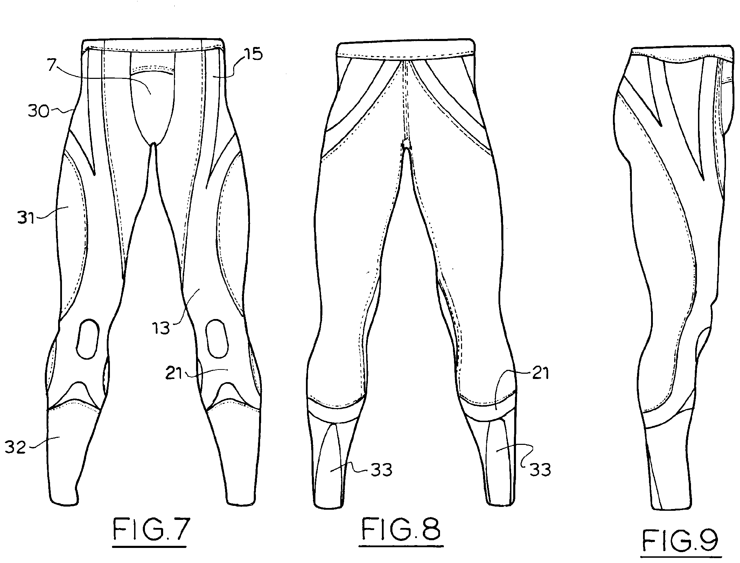

Reference is made to Figs. 7-9, also showing a front, rear and right hand side view of compression garment 1 according to a third preferred embodiment. In this embodiment, the frictive elements 12 and 13 are formed from a single continuous frictive element most preferably a rubber or synthetic rubber material. However, the compression garment outer is formed from a plurality of panels stitched together with a plurality of seams. For example, five portions and waist portions 31 and 30 respectively, are formed from a first type of stretchable material having a first stretchability between 150% and 225%. Crotch panel 7 is formed from a second type of stretchable material having a different stretchability to the panels 30 or 31. Shin panels 32 are also provided about a lower end of each leg of the garment. As best seen in the rear view, garment 1 includes panels adjoining the shin panels 32 corresponding to the locations of the Achilles tendons. These are denoted the Achilles panels 33 and are formed from a stretchable material having a different stretchability to panels 30, 31, 32, and preferably also 7. In a more preferred embodiment, the same stretchable material is used for the crotch as for the Achilles panels. However, it will be appreciated that the stretchability of any panels relative to any other panel can be the same or different as desired.

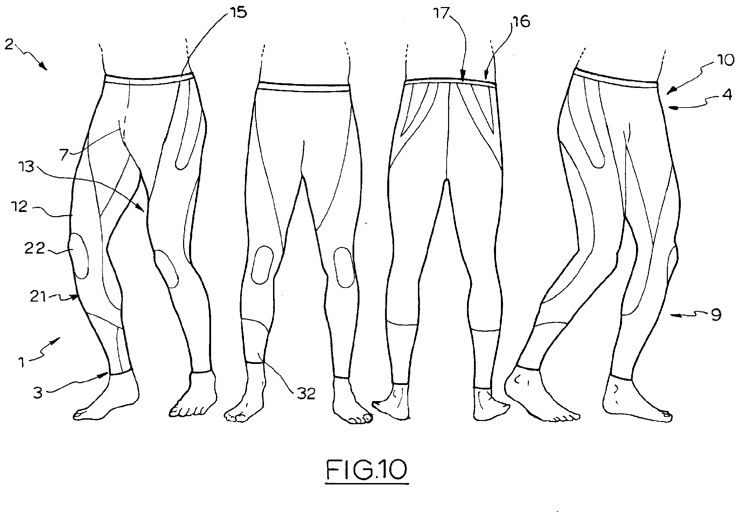

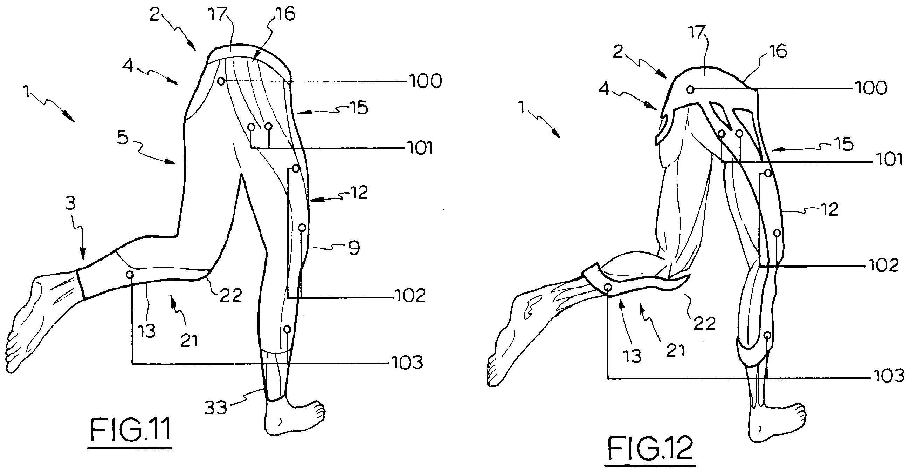

Lastly as illustrated in Figures 10 to 12, the garment can be configured with proprioceptive bands or frictive elements that externally stimulate the proprioceptive sensors in important muscular groups of the lower body, thereby keeping muscles activated and controlled such that the user can exert themselves harder and for longer. The garment is provided with a waist band (100) that is ergonomically shaped and supports the lower abdominal muscles and the lumbar spine without restricting hip rotation or movement.

The upper portion of the bands (101) wrap from the front adductors to the gluteal muscles and iliotibial tract receptors to activate and support these powerful muscles. The middle portion of the bands (102) wrap around the adductor and quadriceps muscle receptors thereby keeping them activated including after the onset of fatigue. The U-shaped portion of the bands (103), principally those that wrap around the lower leg, hug the gastrocnemius muscle receptors to support and protect against involuntary movement and vibration.

The foregoing describes only one embodiment of the present invention and modifications, obvious to those skilled in the compression garment art, can be made thereto without departing from the scope of the present invention, which is solely defined by the appended claims.

## Claims

1. A compression garment (1) extending longitudinally from a waist (2) to at least a distal or lower end of the gastrocnemius or the soleus of each leg of a wearer, the garment comprising:
at least one panel (5, 7) of stretchable material stitched together by a plurality of seams (6, 8) to form a compression garment outer having a waist portion (4) and a pair of leg portions (9) wherein the compression garment outer includes an inside surface facing the user and an external surface , the panel having a warp or longitudinal stretch of between 150% to 225%;
at least one frictive element (12, 13) attached to the inside of the compression garment outer and extending from the waist to the distal or lower end of the gastrocnemius or to the soleus wherein the frictive element has a warp or longitudinal stretch of between 101% to 125%, the frictive element having a plurality of strips (15-17) of predetermined constant or non-constant width;
wherein a first frictive strip (15) extends from the waist about the tensor fasciae latae or sartorius muscle head down to the Sartorius muscle near a top of the vastus medialis, a second frictive strip (16) extending from the waist about the gluteus medius around the vastus intermedius and converging with the first strip over the Sartorius muscle, and a third strip (17) extending from the gluteus maximus at the waist adjacent the spine around the vastus lateralis to converge with the second frictive strip over the iliotibial tract whereby the converged second and third strips extend along the vastus intermedus and converge with the first strip at the sartorius muscle and extending along the rectus femoris a predetermined distance above the patella (22) bifurcating along each side thereof, the frictive element extending a predetermined distance below the patella and around the tibia about the iliotibial tract joining at the distal or lower end of the gastrocnemius muscles or the soleus muscle.

2. A compression garment as claimed in claim 1 wherein the frictive element is glued or stitched to the compression garment outer, or the two are welded or melted together.

3. A compression garment as claimed in any one of claims 1 to 3 wherein the frictive element is formed by a left hand frictive element and a right hand frictive element each extending from the waist to the lower end or bottom of the gastrocnemius or the soleus of each leg respectively.

4. A compression garment as claimed in any one of claims 1 to 4 wherein the frictive element is composed of a natural or synthetic rubber, a polymeric material or an impregnated fabric.

5. A compression garment as claimed in any one of claims 1 to 5 wherein the compression garment outer is formed from a plurality of panels of stretchable material stitched together by a plurality of seams.

6. A compression garment as claimed in claim 5 wherein the compression garment outer includes:
a crotch panel; an Achilles panel; a lower tibia panel; and a pair of upper leg panels.

7. A compression garment as claimed in claim 6 wherein the panels forming the compression garment outer are formed from stretchable material wherein at least two of the panels have different stretchability.

8. A compression garment as claimed in any one of claims 1 to 7 wherein the fictive element includes substantially the same stretchability longitudinally as transversely.

## Patentansprüche

1. Kompressionskleidungsstück (1), das sich longitudinal von einer Taille (2) zu mindestens einem distalen oder unteren Ende des Gastrocnemius oder des Soleus jedes Beins eines Trägers erstreckt, wobei das Kleidungsstück Folgendes umfasst:
mindestens eine Bahn (5, 7) aus dehnbarem Material, die durch mehrere Nähte (6, 8) zusammengenäht ist, um ein Kompressionskleidungsstück-Äußeres mit einem Taillenabschnitt (4) und einem Paar Beinabschnitte (9) zu bilden, wobei das Kompressionskleidungsstück-Äußere eine dem Benutzer zugewandte Innenfläche und eine Außenfläche aufweist, wobei die Bahn eine Kett- oder Längsdehnung zwischen 150 % und 225 % aufweist;
mindestens ein Reibungselement (12, 13), das an der Innenseite des Kompressionskleidungsstück-Äußeren angebracht ist und sich von der Taille zum distalen oder unteren Ende des Gastrocnemius oder zum Soleus erstreckt, wobei das Reibungselement eine Kett- oder Längsdehnung zwischen 101 % und 125 % aufweist, wobei das Reibungselement mehrere Streifen (15-17) mit vorbestimmter konstanter oder nicht konstanter Breite aufweist;
wobei sich ein erster Reibungsstreifen (15) von der Taille um den Tensor fasciae latae oder den Sartorius-Muskelkopf nach unten zum Sartorius-Muskel in der Nähe einer Spitze des Vastus medialis erstreckt, ein zweiter Reibungsstreifen (16) sich von der Taille um den Gluteus medius um den Vastus intermedius erstreckt und mit dem ersten Streifen über dem Sartorius-Muskel konvergiert, und ein dritter Streifen (17) sich vom Gluteus maximus an der Taille nahe der Wirbelsäule um den Vastus lateralis erstreckt, um mit dem zweiten Reibungsstreifen über dem Tractus iliotibialis zu konvergieren, wobei sich der konvergierende zweite und dritte Streifen entlang des Vastus intermedus erstrecken und mit dem ersten Streifen am Musculus sartorius konvergieren und sich entlang des Rectus femoris in einem vorbestimmten Abstand oberhalb der Patella (22) erstrecken und sich entlang jeder Seite davon verzweigen,
wobei sich das Reibungselement in einem vorbestimmten Abstand unterhalb der Patelle und um die Tibia herum um den Tractus iliotibialis erstreckt und sich am distalen oder unteren Ende der Musculi gastrocnemius oder des Musculus soleus vereinigt.

2. Kompressionskleidungsstück nach Anspruch 1, wobei das Reibungselement mit dem Kompressionskleidungsstück-Äußeren verklebt oder vernäht ist, oder die beiden Teile miteinander verschweißt oder verschmolzen sind.

3. Kompressionskleidungsstück nach einem der Ansprüche 1 bis 3, wobei das Reibungselement aus einem linken Reibungselement und einem rechten Reibungselement gebildet ist, die sich jeweils von der Taille bis zum unteren Ende oder Boden des Gastrocnemius oder des Soleus jedes Beins erstrecken.

4. Kompressionskleidungsstück nach einem der Ansprüche 1 bis 4, wobei das Reibungselement aus einem natürlichen oder synthetischen Kautschuk, einem polymeren Material oder einem imprägnierten Gewebe besteht.

5. Kompressionskleidungsstück nach einem der Ansprüche 1 bis 5, wobei das Kompressionskleidungsstück-Äußere aus mehreren Bahnen aus dehnbarem Material gebildet ist, die durch mehrere Nähte zusammengenäht sind.

6. Kompressionskleidungsstück nach Anspruch 5, wobei das Kompressionskleidungsstück-Äußere Folgendes beinhaltet:
eine Schrittbahn; eine Achillesbahn; eine untere Tibiabahn; und ein Paar Oberschenkelbahnen.

7. Kompressionskleidungsstück nach Anspruch 6, wobei die das Kompressionskleidungsstück-Äußere bildenden Bahnen aus dehnbarem Material gebildet sind, wobei mindestens zwei der Bahnen eine unterschiedliche Dehnbarkeit aufweisen.

8. Kompressionskleidungsstück nach einem der Ansprüche 1 bis 7, wobei das Reibungselement longitudinal im Wesentlichen die gleiche Dehnbarkeit hat wie transversal.

## Revendications

1. Vêtement de compression (1) s'étendant longitudinalement depuis une taille (2) jusqu'à au moins une extrémité distale ou inférieure du gastrocnémien ou du soléaire de chaque jambe d'un porteur, le vêtement comprenant :
au moins un panneau (5, 7) de matériau étirable cousu ensemble par une pluralité de coutures (6, 8) pour former un extérieur de vêtement de compression ayant une portion taille (4) et une paire de portions jambes (9) dans lequel l'extérieur de vêtement de compression comporte une surface intérieure faisant face à l'utilisateur et une surface externe, le panneau ayant un allongement ou un étirement longitudinal compris entre 150 % à 225 % ;
au moins un élément à frottement (12, 13) attaché à l'intérieur de l'extérieur de vêtement de compression et s'étendant depuis la taille jusqu'à l'extrémité distale ou inférieure du gastrocnémien ou jusqu'au soléaire dans lequel l'élément à frottement a un allongement ou un étirement longitudinal compris entre 101 % à 125 %, l'élément à frottement ayant une pluralité de bandes (15 à 17) de largeur constante ou non constante prédéterminée ;
dans lequel une première bande à frottement (15) s'étend depuis la taille aux environs du tenseur du fascia lata ou de la tête du muscle couturier vers le bas jusqu'au muscle couturier près d'un sommet du vaste médial, une deuxième bande à frottement (16) s'étendant depuis la taille aux environs du moyen fessier autour du vaste intermédiaire et convergeant avec la première bande au-dessus du muscle couturier, et une troisième bande (17) s'étendant depuis le grand fessier au niveau de la taille à côté de la colonne vertébrale autour du vaste externe pour converger avec la deuxième bande à frottement au-dessus de la bandelette de Maissiat, moyennant quoi les deuxième et troisième bandes convergentes s'étendent le long du vaste intermédiaire et convergent avec la première bande au niveau du muscle couturier et s'étendent le long du droit antérieur sur une distance prédéterminée au-dessus de la rotule (22) en bifurquant le long de chaque côté de celle-ci,
l'élément à frottement s'étendant sur une distance prédéterminée au-dessous de la rotule et autour du tibia aux environs de la bandelette de Maissiat en se rejoignant à l'extrémité distale ou inférieure des muscles gastrocnémiens ou du muscle soléaire.

2. Vêtement de compression selon la revendication 1 dans lequel l'élément à frottement est collé ou cousu sur l'extérieur de vêtement de compression, ou les deux sont soudés ou fusionnés ensemble.

3. Vêtement de compression selon l'une quelconque des revendications 1 à 3 dans lequel l'élément à frottement est formé par un élément à frottement gauche et un élément à frottement droit s'étendant chacun depuis la taille jusqu'à l'extrémité inférieure ou au bas du gastrocnémien ou du soléaire de chaque jambe respectivement.

4. Vêtement de compression selon l'une quelconque des revendications 1 à 4 dans lequel l'élément à frottement est composé d'un caoutchouc naturel ou synthétique, d'un matériau polymère ou d'une étoffe imprégnée.

5. Vêtement de compression selon l'une quelconque des revendications 1 à 5 dans lequel l'extérieur de vêtement de compression est formé à partir d'une pluralité de panneaux de matériau étirable cousus ensemble par une pluralité de coutures.

6. Vêtement de compression selon la revendication 5 dans lequel l'extérieur de vêtement de compression comporte :
un panneau d'entre-jambe ; un panneau de tendon d'Achille ; un panneau de tibia inférieur ; et une paire de panneaux de jambe supérieurs.

7. Vêtement de compression selon la revendication 6 dans lequel les panneaux formant l'extérieur de vêtement de compression sont formés à partir de matériau étirable dans lequel au moins deux des panneaux ont une aptitude à l'étirement différente.

8. Vêtement de compression selon l'une quelconque des revendications 1 à 7 dans lequel l'élément à frottement comporte sensiblement la même aptitude à l'étirement longitudinalement et transversalement.
